# EUROPÄISCHE PATENTANMELDUNG

(11) **EP 0 837 054 A2**
(43) Veröffentlichungstag der Anmeldung: **22.04.1998**
(21) Anmeldenummer: 97116765.5
(22) Anmeldetag: 26.09.1997
(51) Int. Cl.: C07C 255/54, C09K 19/12, C09K 19/20, C09K 19/36, C09K 19/38, C07D 493/04, C07J 9/00, C07C 69/54, C07C 69/92

(54) **Verbindungen und deren Verwendung sowie Verfahren zur Herstellung von flüssigkristallinen Polymeren daraus**

(30) Priorität: 18.10.1996 DE 19643048
(71) Anmelder: Daimler-Benz Aktiengesellschaft, 70546 Stuttgart (DE)
(72) Erfinder: Strohriegl, Peter Dr., 95448 Bayreuth (DE); Strelzyk, Katja, 89075 Ulm (DE); Stohr, Andreas, 65830 Kriftel (DE); Grundig, Petra, 89081 Ulm (DE); Gailberger, Michael, 89231 Neu-Ulm (DE); Dannenhauer, Fritz, 79686 Hasel (DE); Barth, Anne, 89231 Neu-Ulm (DE)

(57) **Zusammenfassung**

Die Erfindung betrifft eine Verbindungen der allgemeinen Formel Y¹-A¹-M¹-A²-Y², worin
- Y¹ und Y² voneinander verschieden sind und Y¹ einen Acrylat- oder Methacrylatrest und Y² einen Vinylether-, Epoxid- oder Azidrest bedeutet,
- A¹ und A² gleiche oder verschiedene Reste der allgemeinen Formel CₙH₂ₙ sind, in der n eine ganze Zahl von 0 bis 20 ist und eine oder mehrere Methylengruppen durch Sauerstoffatome ersetzt sein können, und
- M¹ die allgemeine Formel -R¹-X¹-R²-X²-R³-X³-R⁴- besitzt, wobei
- R¹, R², R³ und R⁴ gleiche oder verschiedene zweibindige Reste aus der Gruppe -O-, -COO-, -CONH-, -CO-, -S-, -C≡C-, -CH=CH-, - CH=N-, -CH₂-, -N=N- und -N=N(O)- bedeuten und R²-X²-R³ oder R²-X²-R³-X³ auch eine C-C-Bindung sein kann und
- X¹, X² und X³ gleiche oder verschiedene Reste aus der Gruppe 1,4-Phenylen, 1,4-Cyclohexylen, mit B¹, B² und/oder B³ substituiertes Arylalkan oder Heteroarylalkan, das 1 bis 3 Heteroatome aus der Gruppe O, N und S enthält, mit 1 bis 10 Kohlenstoffatomen und mit B¹, B² und/oder B³ substituiertes Cycloalkylen mit 1 bis 10 Kohlenstoffatomen sind, worin B¹, B² und B³ gleiche oder verschiedene Substituenten aus der Gruppe -H, C₁-C₂₀-Alkyl, C₁-C₂₀-Alkoxy, C₁-C₂₀-Alkylthio, C₁-C₂₀-Alkylcarbonyl, C₁-C₂₀-Alkoxycarbonyl, C₁-C₂₀-Alkylthiocarbonyl, -OH, Halogen (Fluor, Chlor, Brom, Iod), -CN, -NO₂, Cycloalkyl, Formyl, Acetyl und durch Ethersauerstoff, Thioetherschwefel oder Estergruppen unterbrochene Alkyl-, Alkoxy- oder Alkylthioreste mit 1-20 Kohlenstoffatomen sein können.

## Beschreibung

Polymerbeschichtungen und Effektlacke sind für die Vergütung von Oberflächen und die ästhetische Gestaltung von Gegenständen von großer Bedeutung. Unterschiedlichste Farbeindrücke und besondere Farbeffekte können auf verschiedene Art und Weise erzeugt werden. Gängige Polymerbeschichtungen verwenden zur Farbgebung oder, um bestimmte Effekte zu erzielen, wie beispielsweise Metallicglanz oder ähnliches, Partikel oder Pigmente in einem Trägerpolymer. Um bestimmte Reflexionseffekte zu erreichen, werden beispielsweise Metallflitter, beschichtete Glimmerpartikel oder Interferenzpigmente auf der Basis von flüssigkristallinen Verbindungen in einen klaren Lackkörper als Trägerpolymer eingearbeitet. Dabei können zur freien Gestaltung des Farbeindrucks zusätzlich andere Pigmente beigefügt sein.

Eine andere Möglichkeit zur effektvollen Farbgebung besteht in der Verwendung von flüssigkristallinen Polymeren oder Copolymeren, Oligomeren (Makromonomeren) oder Monomeren. Einige dieser flüssigkristallinen Materialien sind geeignet, zusammenhängende Polymerfilme zu bilden. Sie polymerisieren in der flüssigkristallinen Phase und erzeugen dabei eine Lackschicht mit besonderem Farbeffekt. Das Einarbeiten in ein Trägermaterial, wie beispielsweise in einen klaren Lack, ist nicht erforderlich.

Bekannte Substanzen, die einen flüssigkristallinen Zustand zeigen, sind in der Regel langgestreckte organische Moleküle, die geeignet sind, einen bestimmten molekularen Ordnungszustand einzunehmen. Abhängig von dem Ordnungszustand der flüssigkristallinen Phase werden von einfallendem Licht nur diejenigen Wellenlängen reflektiert, die mit den äquidistanten Netzebenenabständen der flüssigkristallinen Materialien interferieren, wodurch bestimmte Farb- und Reflexionseffekte erzeugt werden. Um Lacke oder andere Polymerbeschichtungen herzustellen, die bestimmte Wellenlängenreflexionen und Lichteffekte aufweisen, ist es erforderlich, die flüssigkristalline Phase zu fixieren bzw. mechanisch zu stabilisieren. Die Ausbildung bestimmter flüssigkristalliner Phasen erfolgt in bestimmten Temperaturbereichen, deren Lage und Breite wiederum vom chemischen Aufbau der Materialien abhängt. Außerdem ist die Farberscheinung der flüssigkristallinen Phasen innerhalb der Phase häufig von der Temperatur abhängig, d. h. es werden beim Erwärmen oder Abkühlen der flüssigkristallinen Phase unterschiedliche Wellenlängen reflektiert. Um einen bestimmten Farb- oder Reflexionseffekt dauerhaft festzuhalten, ist es möglich, eine flüssigkristalline Phase durch Polymerisation bzw. chemische Vernetzung der Ausgangsmoleküle zu einem dichten Netzwerk zu fixieren. Dazu müssen die Ausgangsmaterialien vernetzbare, reaktive Gruppen enthalten.

Aus der Literatur sind flüssigkristalline Monomerverbindungen mit zwei gleichen, endständigen reaktiven Gruppen bekannt, wie Diacrylate (J. Lub, D. J. Broer, R. A. M. Hikmet, K. G. J. Nierop, Liquid Crystals **18**, 319 (1995)), Diepoxide (D. J. Broer, J. Lub, G. N. Mol, Macromolecules **26**, 1244 (1993)) und Divinylether (R. A. M. Hikmet, J. Lub, J. A. Higgins, Polymer **34**, 1736 (1993); S. Jahromi, J. Lub, G. N. Mol, Polymer 35, 622 (1994)). Die Vernetzung solcher Monomere geschieht in der Regel photochemisch durch Photozykloaddition oder unter Zugabe eines Photoinitiators zu dem Monomergemisch. Ebenso sind thermisch initiierte radikalische Vernetzungen oder thermisch initiierte Additions- bzw. Kondensationsreaktionen bekannt. Bei diesen bekannten Monomerverbindungen werden die beiden endständigen Gruppen immer gleichzeitig polymerisiert. Zur Ausbildung eines Polymerfilms werden die bekannten Flüssigkristallmonomere auf die entsprechenden Substrate aufgebracht und die Polymerisationsreaktion initiiert, wobei das fertige Endprodukt entsteht.

Große Schwierigkeiten kann hierbei die Applikation und Haftfähigkeit der Ausgangsmonomere auf dem zu beschichteten Substrat verursachen. Die Flüssigkristallmonomere sind in der Regel kristallin oder pulverförmig, weshalb sie auf dem Untergrund schlecht haften und was den Auftrag in einer gleichmäßigen Schichtdicke erheblich erschwert. Zudem sind die bekannten Flüssigkristallmaterialien hinsichtlich Härte, Elastizität und Haftung des Endprodukts, nämlich des Polymerfilms, relativ invariabel und schlecht auf die Erfodernisse bestimmter Anwendungen einstellbar.

Es ist die Aufgabe der vorliegenden Erfindung, Verbindungen und ein Verfahren zur Herstellung von flüssigkristallinen Polymeren mit gegenüber dem Stand der Technik verbesserten Handhabungs-, Verarbeitungs- und Endprodukteigenschaften zu liefern.

Diese Aufgabe wird durch Verbindungen gelöst mit der allgemeinen Formel Y¹-A¹-M¹-A²-Y², worin
- Y¹ und Y² voneinander verschieden sind und Y¹ einen Acrylat- oder Methacrylatrest und Y² einen Vinylether-, Epoxid- oder Azidrest bedeutet,
- A¹ und A² gleiche oder verschiedene Reste der allgemeinen Formel CₙH₂ₙ sind, in der n eine ganze Zahl von 0 bis 20 ist und eine oder mehrere Methylengruppen durch Sauerstoffatome ersetzt sein können, und
- M¹ die allgemeine Formel -R¹-X¹-R²-X²-R³-X³-R⁴- besitzt, wobei
- R¹, R², R³ und R⁴ gleiche oder verschiedene zweibindige Reste aus der Gruppe -O-, -COO-, -CONH-, -CO-, -S-, -C≡C-, -CH=CH-, - CH=N-, -CH₂-, -N=N- und -N=N(O)- bedeuten und R²-X²-R³ oder R²-X²-R³-X³ auch eine C-C-Bindung sein kann und
- X¹, X² und X³ gleiche oder verschiedene Reste aus der Gruppe 1,4-Phenylen, 1,4-Cyclohexylen, mit B¹, B² und/oder B³ substituiertes Arylalkan oder Heteroarylalkan, das 1 bis 3 Heteroatome aus der Gruppe O, N und S enthält, mit 1 bis 10 Kohlenstoffatomen und mit B¹, B² und/oder B³ substituiertes Cycloalkylen mit 1 bis 10 Kohlenstoffatomen sind, worin B¹, B² und B³ gleiche oder verschiedene Substituenten aus der Gruppe -H, C₁-C₂₀-Alkyl, C₁-C₂₀-Alkoxy, C₁-C₂₀-Alkylthio, C₁-C₂₀-Alkylcarbonyl, C₁-C₂₀-Alkoxycarbonyl, C₁-C₂₀-Alkylthiocarbonyl, -OH, Halogen (Fluor, Chlor, Brom, Iod), -CN, -NO₂, Cycloalkyl, Formyl, Acetyl und durch Ethersauerstoff, Thioetherschwefel oder Estergruppen unterbrochene Alkyl-, Alkoxy- oder Alkylthioreste mit 1-20 Kohlenstoffatomen sein können

Gleichzeitig wird ein Verfahren zur Herstellung von flüssigkristallinen Polymeren bereitgestellt, bei dem
a)durch Polymerisation unter Reaktion der Acrylat- oder Methacrylatgruppen Y¹ einer Verbindung oder einer Mischung von Verbindungen gemäß Anspruch 1 Vorpolymere hergestellt werden und diese danach
b)durch Polymerisation unter Reaktion der Vinylether-, Epoxid- oder Azidgruppen Y² vernetzt werden.

Der besondere Vorteil der erfindungsgemäßen Verbindungen liegt darin, daß sie bei der Polymerisation, insbesondere der Herstellung von flüssigkristallinen Polymeren ein zweistufiges Verfahren ermöglichen. Die vorgeschlagenen Verbindungen besitzen einen molekularen Aufbau, der ihnen flüssigkristalline Eigenschaften verleiht. Zusätzlich besitzen sie polymerisierbare Reste Y¹ und Y², die die Fixierung der polykristallinen Phase durch Polymerisation ermöglichen. Im Gegensatz zu bekannten Flüssigkristallmonomeren, die zur Herstellung von flüssigkristallinen Polymerbeschichtungen einsetzbar sind, sind die unterschiedlichen reaktiven Reste Y¹ und Y² der erfindungsgemäßen Verbindungen jedoch durch Polymerisationsreaktionen mit verschiedenen Initiations- und Reaktionsmechanismen vernetzbar. Durch Polymerisation der Acrylat- oder Methacrylatgruppen Y¹ lassen sich Vorpolymere mit nicht zu hohen Polymerisationsgraden (Oligomere) herstellen. Diese Oligomere bilden Gläser, sind aber noch niedrigviskos genug, um eine gute Orientierung zu gewährleisten. Außerdem lassen sich mit üblichen Methoden der Polymerchemie die Molekulargewichte und andere Materialparameter, wie Viskosität, Filmbildungseigenschaften, Verlauf, Fließverhalten, Löslichkeit, Farbe, Glanz, Quellung, Verarbeitbarkeit, Haftung, Elastizität, Härte usw., beeinflussen.

Eine besonders vorteilhafte Eigenschaft der reaktiven Gruppen Y¹ und Y² der erfindungsgemäßen Verbindungen liegt darin, daß die Initiation und die Reaktion der Vorpolymerisation der reaktiven Gruppen Y¹ stattfinden kann, ohne daß die reaktiven Gruppen Y² dabei zur Reaktion kommen. Die entstehenden Oligomere können im Anschluß an die Vorpolymerisation gut weiterverarbeitet werden und weisen gegenüber den bekannten polykristallinen Monomeren hervorragende Applikationseigenschaften auf. Die Viskosität der Vorpolymere ermöglicht es beispielsweise, diese vor der endgültigen Vernetzung wie einen Lack mit guten Verlaufseigenschaften, gutem Fließverhalten, hoher Spritzsicherheit und ausgezeichneter Haftung auf den zu beschichtenden Untergrund aufzutragen. Im zweiten Polymerisationsschritt werden die noch freien Vinylether-, Epoxid- oder Azidgruppen Y² vernetzt. Die Haftung des Endprodukts auf dem Untergrund kann beispielsweise durch die Konzentration der reaktiven Gruppen Y² dem jeweiligen Substrat angepaßt werden.

Die erfindungsgemäßen Verbindungen weisen noch weitere Vorteile auf. Die Polymerisation der flüssigkristallinen Materialien muß nicht notwendigerweise in zwei Schritten über ein Vorpolymerisat erfolgen. Sie können auch direkt auf einem Substrat über die beiden funktionellen Gruppen Y¹ und Y² polymerisiert werden. Der Anwender hat somit die Möglichkeit zwischen einem einstufigen und einem zweistufigen Polymerisationsverfahren auszuwählen oder diese je nach Anwendung zu kombinieren.

Besonders vorteilhafte Flüssigkristallmonomere sind Verbindungen, bei denen Y¹ eine Acrylat- und Y² eine Vinylethergruppe ist. Zweckmäßig ist es auch, wenn die Anzahl n der Kohlenstoffatome der Reste A¹ und A² 1 bis 10, besonders bevorzugt 2 bis 6, ist. Vorteilhaft für die flüssigkristallinen Eigenschaften ist es, wenn die Reste R¹, R², R³ und/oder R⁴ -O- oder -COO- sind. Zweckmäßig ist es auch, wenn die Reste X¹ und X³ 1,4-Phenylen sind und/oder R²-X²-R³ eine C-C-Bindung ist.

Bei einer bevorzugten Ausführungsform des Verfahrens zur Herstellung von flüssigkristallinen Polymeren erfolgt die Polymerisation (a) unter Reaktion der Acrylat- oder Methacrylatgruppen Y¹ radikalisch und die Polymerisation (b) unter Reaktion der Vinylether-, Epoxid- oder Azidgruppen Y² vorzugsweise photochemisch, besonders zweckmäßig kationisch. Da die Polymerisationsreaktion (a) in der Regel durch Sauerstoff inhibiert wird, ist es zweckmäßig, diese in einem organischen Lösungsmittel, vorzugsweise in entgastem, absolutem Tetrahydrofuran durchzuführen. Geeignet ist es auch, diese Reaktion in Gegenwart eines radikalischen Polymerisationsinitiators, vorzugsweise 2,2*'*-Azobis-(2-methylpropionitril), Dibenzoylperoxid, oder Di-t-butylperoxid, besonders bevorzugt bei einer Initiatorkonzentration von 1 bis 5 Mol-% zu starten bzw. ablaufen zu lassen. Für die Polymerisationsreaktion (a) günstig ist auch das Vorliegen eines Reaktionsreglers, vorzugsweise 1-Dekanthiol, besonders bevorzugt bei einer Reaktionsreglerkonzentration von 1 bis 10 Mol-%.

Die unterschiedlichen Initiations- und Reaktionsmechanismen der radikalischen Polymerisation der Acrylat- oder Methacrylatgruppen Y¹ zum Vorpolymerisat einerseits und der photochemischen, kationischen Polymerisation (b) der Vinylether-, Epoxid- oder Azidgruppen Y² zum Endprodukt andererseits gewährleisten, daß im ersten Schritt bei der Vernetzung der Reste Y¹ keine Reaktionen mit den Resten Y² oder der Reste Y² untereinander stattfinden. Da die kationische Polymerisation im Gegensatz zur radikalischen Polymerisation nicht durch Sauerstoff inhibiert wird, erleichtert dies den verfahrenstechnischen Aufwand und die Handhabung des Vorpolymerisats bei der Vernetzung auf dem Substrat erheblich. Es müssen keinerlei Maßnahmen zum Ausschluß von Sauerstoff durch aufwendige Inertgastechnologien getroffen werden.

Die Durchführung der Vorpolymerisation (a) in einem organischen Lösungsmittel gestattet die Herstellung der gut zu handhabenden Oligomere in großen Mengen und unabhängig von der Stätte der Weiterverarbeitung zum Endprodukt. Die Vorpolymerisate weisen in der Regel eine sehr große Lagerstabilität auf.

Durch die Verwendung eines radikalischen Polymerisationsinitiators und gegebenenfalls die Zugabe eines Reaktionsreglers lassen sich die Reaktionsbedingungen hinsichtlich der Ausbeuten und Produkteigenschaften optimieren.

Für viele Anwendungen ist es besonders zweckmäßig, das Vorpolymerisat nach der Polymerisationsreaktion (a) zu isolieren. Dazu wird es vorzugsweise nach der Polymerisationsreaktion (a) in Hexan gefällt und zweckmäßigerweise anschließend umgefällt und/oder getrocknet.

Für die Applikation und um das Vorpolymerisat wie einen Lack auf das zu beschichtende Substrat auftragen zu können ist es besonders zweckmäßig, es vor der Polymerisation (b) der Vinylether-, Epoxid- oder Azidgruppen in einem organischen Lösungsmittel, bevorzugt in Chloroform oder Tetrahydrofuran, zu lösen und das Lösungsmittel vor der eigentlichen Reaktion zu verdampfen.

Die zu applizierende Form des Vorpolymerisats kann so den vielfältigsten Anwendungserfordernissen angepaßt werden. So kann es beispielsweise auch anstelle der monomeren Verbindungen in dem Lösungsmittel in den Handel gebracht werden, was dem Endverbraucher die Herstellung des Vorpolymerisats und damit Arbeitsschritte, Herstellungsanlagen und Kosten erspart. Weiterhin können Lösungen des Vorpolymerisats in beliebigen Konzentrationen und mit definierten Viskositäts-, Verlaufs-, Benetzungs- und Haftungseigenschaften hergestellt werden. Die Applikation auf das zu beschichtende Substrat kann auf jede geeignete Weise erfolgen. So können Oberflächen beispielsweise besprüht oder bestrichen sowie in die Lösung des Vorpolymerisats getaucht werden. Je nach Erfordernis und verwendetem Lösungsmittel kann dieses bei Raumtemperatur oder erhöhter Temperatur, bei Unterdruck oder im Luftstrom verdampft werden. Hierbei ist zu berücksichtigen, daß die Polymerisationsreaktion (b) bei bestimmten Zusammensetzungen bereits bei erhöhten Temperaturen einsetzt, was für einzelne Anwendungen jedoch auch vorteilhaft sein kann.

Bei einer bevorzugten Ausführungsform der Erfindung wird die Polymerisation (b) unter Reaktion der Vinylether-, Epoxid- oder Azidgruppen in Gegenwart mindestens eines Photoinitiators, vorzugsweise eines kationischen Photoinitiators, durchgeführt. Zweckmäßigerweise liegt der Photoinitiator dabei in einer Menge von 0,5 bis 10 Gew.-%, bevorzugt 1 bis 5 Gew.-%, vor und/oder enthält ein Diaryliumsulfoniumsalz, ein Diaryliodoniumsalz oder ein Gemisch davon. Beispiele für derartige Photoinitiatoren sind die handelsüblichen Produkte Degacure KI 85 (Degussa), Bis-(4-tert.butylphenyl)-iodonium-hexafluorophosphat (Midori Chemical), 2,4,6-Trimethylbenzoylethoxyphenylphosphinoxid (BASF) im Gemisch mit Diphenyliodoniumhexafluorophosphat und 2,2-Dimethoxy-2-phenylacetophenon im Gemisch mit Diphenyliodonium-tetrafluoroborat. Normalerweise wird die Polymerisationsreaktion (b) durch ultraviolette Strahlung induziert und/oder das Reaktionsprodukt durch thermische Behandlung nachgehärtet.

Die Verwendung eines Photoinitiators bietet besondere Vorteile, da damit der Reaktionsablauf zum Endprodukt leichter in Gang gebracht, reguliert und beschleunigt werden kann. Gegenüber der rein thermischen Härtung der Vorpolymerisate spart die Induktion der Polymerisationsreaktion durch ultraviolette Strahlung erhebliche Energiekosten ein und ist schneller und zielgerichteter einsetzbar und besser zu dosieren. Die thermische Behandlung hat Vorteile, wenn die zu beschichtende Oberfläche aufgrund von Schattenwurf oder Unzugänglichkeit von der Initiationsstrahlung nicht erreicht werden kann.

Bei einer besonders bevorzugten Ausführungsform des Verfahrens der vorliegenden Erfindung wird die Polymerisation (a) in Gegenwart von zusätzlichen Verbindungen durchgeführt mit der allgemeinen Formel Y³-A³-M²-A⁴-Y⁴ oder (Y³-A³)₂-M²-A⁴-Y⁴, worin
- Y³ eine Acrylat- oder Methacrylatgruppe und Y⁴ ein polymerisierbarer Rest aus der Gruppe der Vinylether-, Epoxid- und Azidreste oder ein nicht polymerisierbarer Rest aus der Gruppe -H, -CN und Cholesteryl sein kann,
- A³ und A⁴ gleiche oder verschiedene Reste der allgemeinen Formel CₙH₂ₙ sind, in der n eine ganze Zahl von 0 bis 20 ist und eine oder mehrere Methylengruppen durch Sauerstoffatome ersetzt sein können, und
- M² die allgemeine Formel -R⁵-X⁴-R⁶-X⁵-R⁷-X⁶-R⁸- besitzt, worin
- R⁵, R⁶, R⁷ und R⁸ gleiche oder verschiedene zweibindige Reste aus der Gruppe -O-, -COO-, -CONH-, -CO-, -S-, -C≡C-, -CH=CH-, - CH=N-, -CH₂-, -N=N- und -N=N(O)- sind und R⁶-X⁵-R⁷ oder R⁶-X⁵-R⁷-X⁶ auch eine C-C-Bindung sein kann und
- X⁴, X⁵ und X⁶ gleiche oder verschiedene Reste aus der Gruppe 1,4-Phenylen, 1,4-Cyclohexylen, mit B¹, B² und/oder B³ substituiertes Arylalkan oder Heteroarylalkan, das 1 bis 3 Heteroatome aus der Gruppe O, N und S enthält, mit 1 bis 10 Kohlenstoffatomen und mit B¹, B² und/oder B³ substituiertes Cycloalkylen mit 1 bis 10 Kohlenstoffatomen sind, worin B¹, B² und B³ gleiche oder verschiedene Substituenten aus der Gruppe -H, C₁-C₂₀-Alkyl, C₁-C₂₀-Alkoxy, C₁-C₂₀-Alkylthio, C₁-C₂₀-Alkylcarbonyl, C₁-C₂₀-Alkoxycarbonyl, C₁-C₂₀-Alkylthiocarbonyl, -OH, Halogen (Fluor, Chlor, Brom, Iod), -CN, -NO₂, Cycloalkyl, Formyl, Acetyl und durch Ethersauerstoff, Thioetherschwefel oder Estergruppen unterbrochene Alkyl-, Alkoxy- oder Alkylthioreste mit 1-20 Kohlenstoffatomen sein können.

Diese zusätzlichen Verbindungen, im folgenden als Comonomere bezeichnet, sind besonders geeignet, die Eigenschaften des Polymerisationsendprodukts den entsprechenden Anwendungserfordernissen anzupassen. Die einstellbaren, mechanischen Eigenschaften des Bndprodukts sind insbesondere Haftfähigkeit, Elastizität und Härte des Polymerfilms auf dem entsprechenden Untergrund.

Besonders vorteilhaft ist die erfindungsgemäße Verwendung der Comonomere zur Beeinflussung des optischen Effekts des Polymerfilms. Dabei ist es besonders zweckmäßig, wenn die Reste A³ und/oder A⁴ chiral sind. Durch die Verwendung von einem oder mehreren chiralen oder chiral-nematischen Comonomeren in unterschiedlichen Mischungsverhältnissen mit den Monomeren der vorliegenden Erfindung können beliebige Reflektionswellenlängen der Polymerfilme vom ultravioletten bis zum infraroten Bereich eingestellt werden. Durch Copolymerisation mit den chiralen Verbindungen entstehen hochvernetzte Polymerfilme, in denen die Reflexionswellenlänge nicht von der Temperatur abhängt. Aufgrund der Chiralität der Verbindungen entstehen bei der Polymerisation (a) cholesterische Seitengruppenvorpolymere, die anschließend in der Polymerisationsreaktion (b) als cholesterisches Netzwerk fixiert werden.

Beispiele für erfindungsgemäß geeignete Comonomere sind folgende Verbindungen:
4-[(S)-(2-Acryloyloxy-2-methyl)-ethyloxy]-4*'*-cyanobiphenyl
   (Monomer 2) 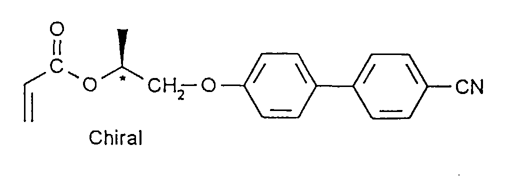
4-[(S)-(2-Acryloyloxy-2-methyl)-ethyloxy]-4*'*-[(S)-2-methylbutyloxy]-biphenyl
   (Monomer 3) 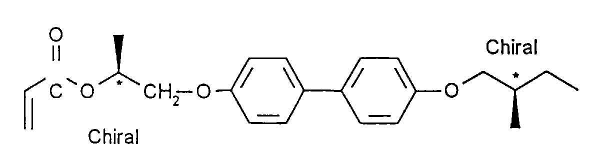
{4*'*-[(S)-(2-Acryloyloxy-2-methyl)-ethyloxy]-phenyl}-4-(2-vinyloxyethoxy)-benzoat
   (Monomer 4)

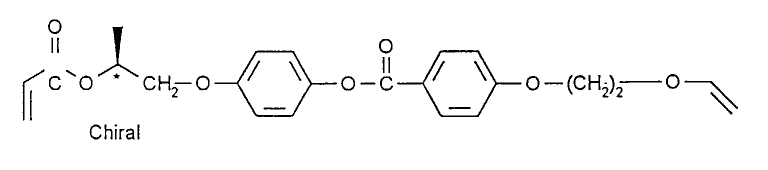

Bevorzugt weisen die Copolymere nur einen oder zwei polymerisierbare Reste Y³ auf, die ausschließlich an der ersten Polymerisationsreaktion (a) beteiligt sind. Copolymere mit nur einer Acrylat- oder Methacrylatgruppe beeinflussen neben dem optischen Effekt auch die mechanischen Eigenschaften des Vorpolymerisats und des Endprodukts. Derartige Copolymere führen bei der Polymerisationsreaktion zu Kettenabbrüchen und regulieren so den Vernetzungsgrad. Die chiralen Zentren A³ und/oder A⁴ der Comonomere begünstigen die Ausbildung einer cholesterischen Phase in dem flüssigkristallinen Polymer. Eine solche cholesterische Struktur, die nur mit optisch aktiven Molekülen realisierbar ist und eine Art Überstruktur der einfach gebauten, flüssigkristallinen Texturen darstellt, enthält flüssigkristalline Elementarbereiche in einer schraubenförmigen, helikalen Anordnung. Die Höhe der einzelnen helikalen Elementarbereiche, auch als Ganghöhe bezeichnet, bestimmt die Wellenlänge des reflektierten Lichtes und damit die Farben der metallisch schillernden Reflexionen des Polymerfilms.

Beispiele für chirale Vinylether und Divinylether, die als erfindungsgemäße Comonomere mit freien Vinylethergruppen geeignet sind und eine cholesterische Phase begünstigen, sind folgende Verbindungen:
Cholesteryl-4-(2-vinyloxyethoxy)benzoat 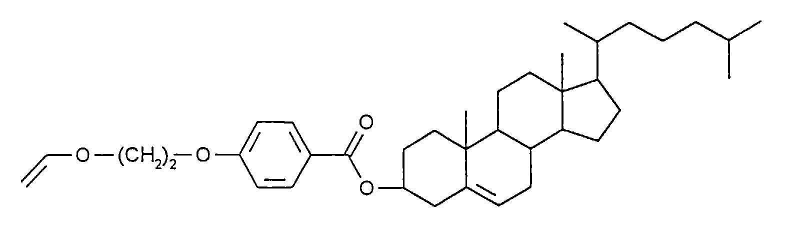
Cholesteryl-4-(6-acryloyloxyhexyloxy)-benzoat (Monomer 5) 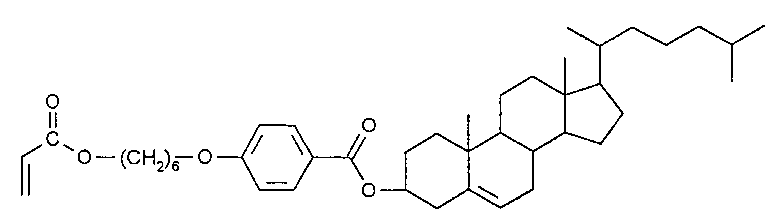
Cholesteryl-3,4-di-(1-vinyloxyethoxy)benzoat 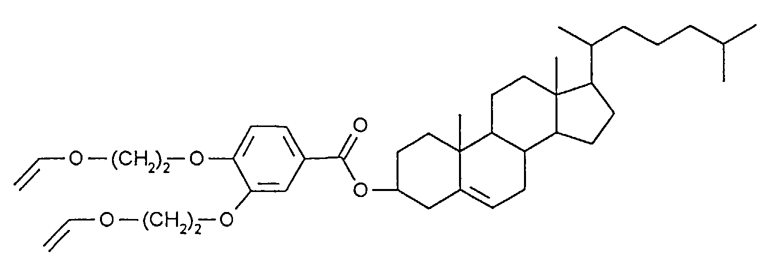
Derivat von 1,4 : 3,6-Dianhydro-D-mannit mit zwei Vinylethergruppen

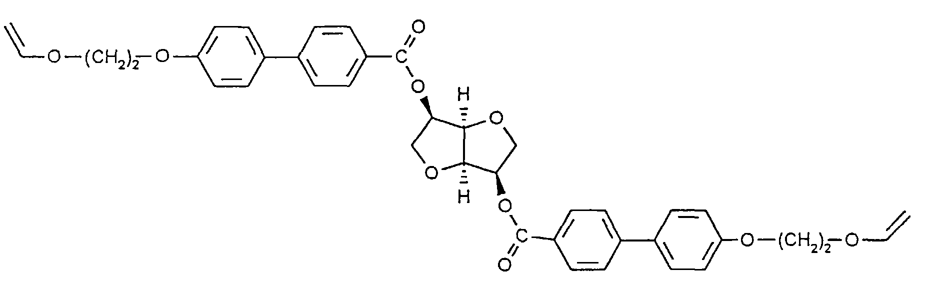

Weitere Vorteile, Merkmale und Anwendungsmöglichkeiten der vorliegenden Erfindung werden deutlich anhand der folgenden Beschreibung bevorzugter Ausführungsbeispiele.

### Beispiel 1

### A)4-(2-Vinyloxyethoxy)benzoesäure-4'-(6-acryloyloxy-hexyloxy)phenylester (Monomer 1)

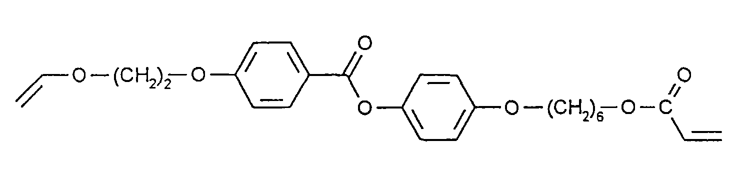

4,37 g (0,021 Mol) 4-(2-Vinyloxyethoxy)benzoesäure werden in 80 ml 1,2-Dimethoxyethan gelöst und 4,25 g (0,042 Mol) Triethylamin zugegeben. Bei -30 °C werden 2,41 g (0,021 Mol) Methansulfochlorid so zu der Lösung getropft, daß die Temperatur nicht über -25 °C ansteigt. Nach 1h Rühren bei -30 °C fügt man 5,56 g (0,021 Mol) 4-(6-Acryloyloxy-hexyloxy)phenol, 0,24 g (0,002 Mol) 4-Dimethylaminopyridin, sowie 100 mg 2,6-Di-tert.butyl-p-kresol zu der Reaktionslösung hinzu und läßt noch 3 h bei 0 bis 5 °C rühren. Danach wird der entstandene Niederschlag abfiltriert und das Filtrat im Vakuum einrotiert. Der Rückstand wird in 80 ml Isopropanol umkristallisiert. Man erhält das Produkt mit einer Ausbeute von 78 % (7,42 g).

### B) Synthese der Vorstufen

a) 4-(2-Vinyloxyethoxy)-benzoesäure 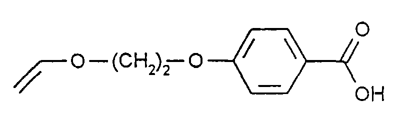
   11,3 g (0,068 Mol) 4-Hydroxybenzoesäure-ethylester werden in 200 ml Cyclohexanon unter Schutzgasatmosphäre gelöst. Nach Zugabe von 18,8 g (0,136 Mol) Kaliumkarbonat, 1,0 g Kaliumiodid und 8,0 g (0,075 Mol) 2-Chlorethyl-vinylether wird das Reaktionsgemisch 5 h unter Rückfluß gekocht. Anschließend wird der Feststoff abfiltriert und mit etwas Cyclohaxanon nachgewaschen.
   Nach Abziehen des Cyclohexanons im Vakuum nimmt man den Rückstand in 300 ml Methanol auf und fügt eine Lösung von 19,04 g (0,34 Mol) KOH in 50 ml Wasser hinzu. Das Reaktionsgemisch wird dann 17 h unter Rückfluß erhitzt. Anschließend wird das Lösungsmittel im Vakuum abgezogen und der Rückstand mit 300 ml 0,5 M wäßriger NaOH aufgenommen. Nach 3-maligem Waschen mit je 80 ml Diethylether wird die wäßrige Phase mit konz. HCl angesäuert, der entstandene Niederschlag abfiltriert und nach dem Trocknen in 100 ml Toluol umkristallisiert. Die Ausbeute beträgt 9,49 g (67 %).
b) Die Synthese von 4-(6-Acryoyloxy-hexyloxy)phenol 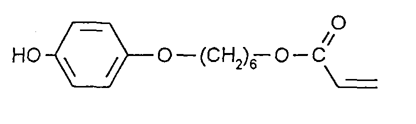 erfolgt nach R. A. M. Hikmet, J. Lub, A. J. W. Tol, Macromolecules **28**, 3313 (1995).

### Beispiel 2

### 4-[(S)-(2-Acryloyloxy-2-methyl)-ethyloxy]-4'-cyanobiphenyl (Monomer 2 gemäß Anspruch 16)

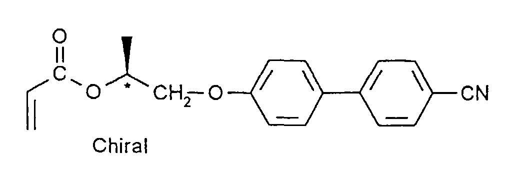

Die Verbindung wird analog dem entsprechenden Methacrylat synthetisiert, wie bei J. M. G. Cowie, H. W. Hunter, Makromol. Chem. **191**, 1339 (1990) beschrieben.

### Beispiel 3

### Synthese eines Oligoacrylates mit freien Vinylethergruppen

1,0 g des Monomeren 1 wird in 10 ml absolutem THF gelöst. Zu der Lösung fügt man 2 Mol-% 2,2*'*-Azobis-(2-methylpropionitril) als Initiator und 4 Mol-% 1-Decanthiol als Regler. Nach 3-maligem Entgasen wird 48 h bei 60 °C unter Rühren polymerisiert.

Die Lösung wird dann in 400 ml Hexan gefällt, das Polymer in 8 ml CHCl₃ gelöst und in 400 ml Methanol umgefällt. Die Ausbeute beträgt nach der Trocknung im Vakuumschrank bei 50 °C über Nach 85 %.

### Charakterisierung:

Das Polymer zeigt eine nematische Phase mit einem Klärpunkt bei ca. 100 °C. Im ¹H-NMR sind die Vinyletherbanden bei 6,5 ppm und 4,2 ppm noch erkennbar. Nach GPC beträgt Mₙ ^{∼} 20000.

### Beispiel 4

### Synthese von cholesterischen Cooligoacrylaten mit freien Vinylethergruppen

1,0 g einer Mischung der Monomeren 1 und 2 werden in 10 ml absolutem THF gelöst. Zu der Lösung fügt man 2 Mol-% 2,2*'*-Azobis-(2-methylpropionitril) als Initiator und 4 Mol-% 1-Decanthiol als Regler hinzu. Nach 3-maligem Entgasen wird 48 h bei 60 °C unter Rühren polymerisiert.

Die Lösung wird dann in 400 ml Hexan gefällt, das Polymer in 8 ml CHCl₃ gelöst und in 400 ml Methanol umgefällt. Anschließend wird im Vakuumschrank bei 50 °C über Nacht getrocknet.

Man erhält ein Cooligoacrylat mit noch freien Vinylethergruppen. Mit diesen läßt sich das Polymer anschließend kationisch vernetzen.

### Beispiel 5

### Kationische Photovernetzung von Filmen der Cooligoacrylate

Das vorpolymerisierte cholesterische Cooligoacrylat mit freien Vinylethergruppen wird in CHCl₃ oder THF gelöst. Nach Zugabe von 1 bis 5 Gew.-% kationischem Photoinitiator Dagacure KI 85 (Degussa) wird die Lösung auf ein Substrat aufgerakelt und das Lösungsmittel verdampft. Die Vorpolymerschicht wird anschließend in der cholesterischen Phase durch Bestrahlung mit UV-Licht kationisch vernetzt. Nach dem Bestrahlen wird der Film völlig unlöslich und ändert seine Textur nicht mehr. Abhängig vom Mischungsverhältnis der Monomeren 1 und 2 liegt die Reflexionswellenlänge der Polymerbeschichtung im sichtbaren Spektralbereich, weshalb der Film farbig und metallisch schillernd erscheint.

### Beispiel 6

### 4-[(S)-(2-Acryloyloxy-2-methyl)-ethyloxy]-4'-[(S)-2-methylbutyloxy]-biphenyl (Monomer 3 gemäß Anspruch 16)

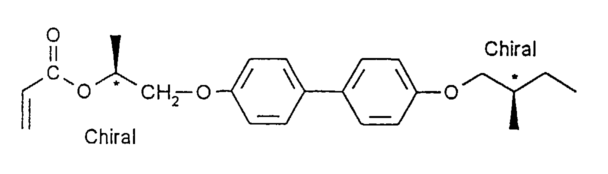

Die chirale Vorstufe 4-Hydroxy-[4'-2-methyl]butyloxy]biphenyl von Monomer 3 wird nach R. Zentel, G. Reckert, B. Reck, Liquid Crystals **2**, 83 (1987) synthetisiert. Die weitere Synthese zum Monomer 3 erfolgt analog der Synthese von Monomer 2.

### Beispiel 7

### Synthese von cholesterischen Cooligoacrylaten mit freien Vinylethergruppen

1,0 g einer Mischung der Monomeren 1 und 3 werden in 10 ml absolutem THF gelöst. Zu der Lösung fügt man 2 Mol-% 2,2*'*-Azobis-(2-methylpropionitril) als Initiator und 4 Mol-% 1-Decanthiol als Regler hinzu. Nach 3-maligem Entgasen wird 48 h bei 60 °C unter Rühren polymerisiert.

Die Lösung wird dann in 400 ml Hexan gefällt, das Polymer in 8 ml CHCl₃ gelöst und in 400 ml Methanol umgefällt. Anschließend wird im Vakuumschrank bei 50 °C über Nacht getrocknet.

Man erhält ein Cooligoacrylat mit noch freien Vinylethergruppen. Mit diesen läßt sich das Polymer anschließend kationisch vernetzen.

### Beispiel 8

### Kationische Photovernetzung von Filmen der Cooligoacrylate

Das vorpolymerisierte cholesterische Cooligoacrylat mit freien Vinylethergruppen wird in CHCl₃ oder THF gelöst. Nach Zugabe von 1 bis 5 Gew.-% kationischem Photoinitiator Dagacure KI 85 (Degussa) wird die Lösung auf ein Substrat aufgerakelt und das Lösungsmittel verdampft. Die Vorpolymerschicht wird anschließend in der cholesterischen Phase durch Bestrahlung mit UV-Licht kationisch vernetzt. Nach dem Bestrahlen wird der Film völlig unlöslich und ändert seine Textur nicht mehr. Abhängig vom Mischungsverhältnis der Monomeren 1 und 3 liegt die Reflexionswellenlänge der Polymerbeschichtung im sichtbaren Spektralbereich, weshalb der Film farbig und metallisch schillernd erscheint.

### Beispiel 9

### A) Cholesteryl-4-(6-acryloyloxyhexyloxy)benzoat (Monomer 5)

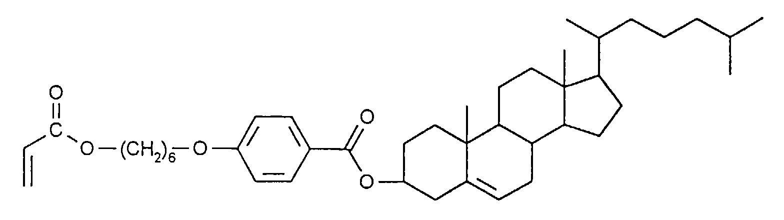

2,92 g (0,01 Mol) 4-(6-Acryloyloxyhexyloxy)benzoesäure werden in 10 ml CHCl₃ gelöst, 2 Tropfen N,N-Dimethylformamid und 30 mg 2,6-Di-tert.-butyl-p-kresol zugesetzt. Anschließend tropft man zu der Lösung 15 ml Thionylchlorid und läßt bei 70 °C rühren bis die Gasentwicklung aufhört (ca. 1 - 2 h). Danach werden das CHCl₃ und überschüssiges Thionylchlorid im Vakuum abdestilliert. Als Rückstand bleibt 4-(6-Acryloyloxyhexyloxy)benzoesäurechlorid.

Das Säurechlorid wird in 30 ml absolutem Ether gelöst und bei 0 bis 5 °C zu 3,86 g (0,01 Mol) Cholesterin in 10 ml Pyridin getropft. Anschließend läßt man 17 h bei 50 °C rühren.

Zur Aufarbeitung wird die Lösung in 200 bis 300 ml Eiswasser gegossen und mit konz. HCl angesäuert. Das Produkt fällt dabei als weißer Niederschlag aus. Der Niederschlag wird abfiltriert, mit gesättigter NaHCO₃-Lösung und danach mit H₂O gewaschen. Anschließend wird 2 mal aus je 100 ml Ethanol umkristallisiert. Nach dem Trocknen erhält man 4,5 g (69 %) weiße Kristalle 27.

### Charakterisierung:

¹H-NMR(CDCl₃): 0,65 (s, 3H); 0,8 bis 2,1 (m, 46 H); 2,45 (m, 2H); 3,88 (t, 2H); 4,15 (t, 2H); 4,821 (m, 1H); 5,40 (m, 1H); 5,80 (dd, 1H); 6,10 (dd, 1H); 6,38 (dd, 1H); 6,85 (d, 2H); 7,95 (d, 2H) ppm
¹³C-NMR(CDCl₃): 11,82; 18,68; 19,34; 21,01; 22,52; 22,78; 23,80; 24,24; 25,66; 27,95; 28,19; 28,49; 28,96; 31,85; 35,75; 36,15; 36,60; 37,01; 38,25; 39,47; 39,70; 42,27; 50,00; 56,10; 56,64; 64,39; 67,87; 74,12; 113,89; 122,59; 123,05; 128,52; 130,44; 131,46; 139,71; 162,66; 165,69; 166,20 ppm.
IR (Kbr): 2943; 2866; 1717; 1634; 1605; 1510; 1273; 1250; 1198; 1167; 1115; 1007; 772 cm⁻¹
Phasenverhalten^{a)}: k97 s 161 ch 200 i
^{a)}Polarisationsmikroskop:
   Heizrate 10 Kmin⁻¹; 2. Aufheizen; 1 Gew.-% 4-Methoxyphenol als Inhibitor. Es sind keine DSC-Daten erhältlich, da die Verbindung durchpolymerisiert. Im Polarisationsmikroskop kann man an den Probenrändern die Phasenübergänge beobachten.

### B)Synthese der Vorstufen

### a) 4-(6-Hydroxyhexyloxy)benzoesäureethylester

16,6 g (0,1 Mol) 4-Hydroxybenzoesäureethylester werden zu einer Lösung von 4,0 g (0,12 Mol) NaOH in 300 ml 2-Butanon gegeben. Nach Zugabe von 15,0 g (0,1 Mol) NaI und 16,01 ml (0,12 Mol) 6-Chlorhexanol wird die Mischung 10 h lang bei 60 °C gerührt.

Das Lösungsmittel wird im Wasserstrahlvakuum (WV) abdestilliert, der Rückstand mit 300 ml 0,4 M NaOH gewaschen und 4 mal mit je 80 ml Ether extrahiert. Die Etherphasen werden vereinigt, über Na₂SO₄ getrocknet und anschließend der Ether im Wasserstrahlvakuum abrotiert.

Als Rückstand bleibt als gelber Feststoff 4-(6-Hydroxyhexyloxy)benzoesäureethylester, der ohne weitere Reinigung eingesetzt wird.
Ausbeute: 23,5 g (88 %)

### b) 4-(6-Hydroxyhexyloxy)benzoesäure

23,5 g (0,088 Mol) 4-(6-Hydroxyhexyloxy)benzoesäureethylester (als Rohprodukt) werden zu 300 ml 0,5 M KOH gegeben und 5 h lang unter Rückfluß gekocht.

Die Lösung wird anschließend 3 mal mit je 80 ml Ether gewaschen und die Wasserphase mit HCl angesäuert. Das ausgefallene Produkt wird abfiltriert, mit H₂O gewaschen und in 250 ml Ethanol umkristallisiert.

Nach dem Trocknen im Vakuum erhält man 9,7 g (50 %) weiße Kristalle.

### Charakterisierung:

¹H-NMR(THF-dg): 1,65 (m, 6H); 1,95 (m, 2H); 3,65 (t, 2H); 4,20 (t, 2H); 7,10 (d, 2H); 8,10 (d, 2H) ppm
IR(KBr): 3404; 2947; 2911; 1690; 1605 cm⁻¹

### c) 4-(6-Acryloyloxyhexyloxy)benzoesäure

16,0 g (0,067 Mol) 4-(6-Hydroxyhexyloxy)benzoesäure werden zu einer Lösung von 9,34 ml (0,074 Mol) N,N-Dimethylanilin in 90 ml 1,4-Dioxan gegeben.

Bei 60 °C werden anschließend 6,01 ml (0,074 Mol) frisch destilliertes Acrylsäurechlorid langsam zugetropft. Danach läßt man noch 2 h lang bei 60 °C rühren. Bei Zugabe von Wasser fällt das Produkt als weißer Niederschlag aus und wird abfiltriert.

Zur Reinigung wird das Produkt in 200 ml 2-Propanol umkristallisiert. Nach dem Trocknen erhält man 14,6 g (75 %) weiße Kristalle.

### Charakterisierung:

¹H-NMR(CDCl₃): 1,45 (m, 4H); 1,70 (M, 2H); 1,80 (m, 2H); 4,00 (t, 2H); 4,15 (t, 2H); 5,80 (dd, 1H); 6,10 (dd, 1H); 6,38 (dd, 1H); 6,90 (d, 2H); 8,05 (d, 2H) ppm
IR(KBr): 2940; 1730; 1688; 1607; 1431; 1410; 1315; 1296; 1198; 1169; 986; 770 cm⁻¹

## Patentansprüche

1. Verbindungen der allgemeinen Formel Y¹-A¹-M¹-A²-Y², worin
- Y¹ und Y² voneinander verschieden sind und Y¹ einen Acrylat- oder Methacrylatrest und Y² einen Vinylether-, Epoxid- oder Azidrest bedeutet,
- A¹ und A² gleiche oder verschiedene Reste der allgemeinen Formel CₙH₂ₙ sind, in der n eine ganze Zahl von 0 bis 20 ist und eine oder mehrere Methylengruppen durch Sauerstoffatome ersetzt sein können, und
- M¹ die allgemeine Formel -R¹-X¹-R²-X²-R³-X³-R⁴- besitzt, wobei
- R¹, R², R³ und R⁴ gleiche oder verschiedene zweibindige Reste aus der Gruppe -O-, -COO-, -CONH-, -CO-, -S-, -C°C-, -CH=CH-, -CH=N-, -CH₂-, -N=N- und -N=N(O)- bedeuten und R²-X²-R³ oder R²-X²-R³-X³ auch eine C-C-Bindung sein kann, wobei n in A¹ bzw. A² eine ganze Zahl von 1 bis 20 ist, wenn R¹ bzw. R⁴ -O- ist, und
- X¹, X² und X³ gleiche oder verschiedene Reste aus der Gruppe 1,4-Phenylen, 1,4-Cyclohexylen, mit B¹, B² und/oder B³ substituiertes Arylalkan oder Heteroarylalkan, das 1 bis 3 Heteroatome aus der Gruppe O, N und S enthält, mit 6 bis 10 Atomen im Arylkern und mit B¹, B² und/oder B³ substituiertes Cycloalkylen mit 3 bis 10 Kohlenstoffatomen sind, worin B¹, B² und B³ gleiche oder verschiedene Substituenten aus der Gruppe -H, C₁-C₂₀-Alkyl, C₁-C₂₀-Alkoxy, C₁-C₂₀-Alkylthio, C₁-C₂₀-Alkylcarbonyl, C₁-C₂₀-Alkoxycarbonyl, C₁-C₂₀-Alkylthiocarbonyl, -OH, Halogen (Fluor, Chlor, Brom, Iod), -CN, -NO₂, Cycloalkyl, Formyl und durch Ethersauerstoff, Thioetherschwefel oder Estergruppen unterbrochene Alkyl-, Alkoxy- oder Alkylthioreste mit 1-20 Kohlenstoffatomen sein können.

2. Verbindungen nach Anspruch 1, dadurch gekennzeichnet, daß Y¹ eine Acrylat- und Y² eine Vinylethergruppe ist.

3. Verbindungen nach einem der Ansprüche 1 oder 2, dadurch gekennzeichnet, daß n eine ganze Zahl von 1 bis 10, bevorzugt von 2 bis 6, ist.

4. Verbindungen nach einem der Ansprüche 1 bis 3, dadurch gekennzeichnet, daß die Reste R¹, R², R³ und/oder R⁴ -O- oder - COO- sind.

5. Verbindungen nach einem der Ansprüche 1 bis 4, dadurch gekennzeichnet, daß die Reste X¹ und X³ 1,4-Phenylen sind und/oder R²-X²-R³ eine C-C-Bindung ist.

6. Verfahren zur Herstellung von flüssigkristallinen Polymeren, dadurch gekennzeichnet, daß
a) durch Polymerisation unter Reaktion der Acrylat- oder Methacrylatgruppen Y¹ einer Verbindung oder einer Mischung von Verbindungen gemäß Anspruch 1 Vorpolymere hergestellt werden und diese danach
b) durch Polymerisation unter Reaktion der Vinylether-, Epoxid- oder Azidgruppen Y² vernetzt werden.

7. Verfahren nach Anspruch 6, dadurch gekennzeichnet, daß die Polymerisation (a) unter Reaktion der Acrylat- oder Methacrylatgruppe Y¹ radikalisch erfolgt oder induziert wird.

8. Verfahren nach Anspruch 6 oder 7, dadurch gekennzeichnet, daß die Polymerisation (b) unter Reaktion der Vinylether-, Epoxid- oder Azidgruppen Y² photochemisch, vorzugsweise kationisch erfolgt.

9. Verfahren nach einem der Ansprüche 6 bis 8, dadurch gekennzeichnet, daß die Polymerisation (a) der Acrylat- oder Methacrylatgruppen Y¹ in einem organischen Lösungsmittel, vorzugsweise in entgastem, absolutem Tetrahydrofuran
und/oder in Gegenwart eines radikalischen Polymerisationsinitiators, vorzugsweise 2,2*'*-Azobis-(2-methylpropionitril), Dibenzoylperoxid, oder Di-t-butylperoxid, besonders bevorzugt bei einer Initiatorkonzentration von 1 bis 5 Mol-%,
und/oder in Gegenwart eines Reaktionsreglers, vorzugsweise 1-Decanthiol, besonders bevorzugt bei einer Reaktionsreglerkonzentration von 1 bis 10 Mol-%, durchgeführt wird.

10. Verfahren nach einem der Ansprüche 6 bis 9, dadurch gekennzeichnet, daß das Vorpolymerisat nach der Polymerisationsreaktion (a) isoliert wird.

11. Verfahren nach einem der Ansprüche 6 bis 10, dadurch gekennzeichnet, daß das Vorpolymerisat nach der Polymerisationsreaktion (a) vorzugsweise in Hexan gefällt und besonders bevorzugt anschließend umgefällt und/oder getrocknet wird.

12. Verfahren nach einem der Ansprüche 6 bis 11, dadurch gekennzeichnet, daß das Vorpolymerisat vor der Polymerisation (b) der Vinylether-, Epoxid- oder Azidgruppen in einem organischen Lösungsmittel, bevorzugt in Chloroform oder Tetrahydrofuran, gelöst und das Lösungsmittel vor der Reaktion verdampft wird.

13. Verfahren nach einem der Ansprüche 6 bis 12, dadurch gekennzeichnet, daß die Polymerisation (b) der Vinylether-, Epoxid- oder Azidgruppen in Gegenwart mindestens eines Photoinitiators, vorzugsweise eines kationischen Photoinitiators, durchgeführt wird.

14. Verfahren nach Anspruch 13, dadurch gekennzeichnet, daß der Photoinitiator in einer Menge von 0,5 bis 10 Gew.-%, bevorzugt 1 bis 5 Gew.-%, vorliegt und/oder ein Diaryliumsulfoniumsalz, ein Diaryliodoniumsalz oder ein Gemisch davon enthält.

15. Verfahren nach einem der Ansprüche 6 bis 14, dadurch gekennzeichnet, daß die Polymerisationsreaktion (b) durch ultraviolette Strahlung induziert wird und/oder das Reaktionsprodukt durch thermische Behandlung nachgehärtet wird.

16. Verfahren nach einem der Ansprüche 6 bis 15, dadurch gekennzeichnet, daß die Polymerisation (a) in Gegenwart von zusätzlichen Verbindungen der allgemeinen Formel Y³-A³-M²-A⁴-Y⁴ oder (Y³-A³)₂-M-A⁴-Y⁴ durchgeführt wird, worin
- Y³ eine Acrylat- oder Methacrylatgruppe und Y⁴ ein polymerisierbarer Rest aus der Gruppe der Vinylether-, Epoxid- und Azidreste oder ein nicht polymerisierbarer Rest aus der Gruppe -H, -CN und Cholesteryl sein kann,
- A³ und A⁴ gleiche oder verschiedene Reste der allgemeinen Formel CₙH₂ₙ sind, in der n eine ganze Zahl von 0 bis 20 ist und eine oder mehrere Methylengruppen durch Sauerstoffatome ersetzt sein können, und
- M² die allgemeine Formel -R⁵-X⁴-R⁶-X⁵-R⁷-X⁶-R⁸- besitzt, worin
- R⁵, R⁶, R⁷ und R⁸ gleiche oder verschiedene zweibindige Reste aus der Gruppe -O-, -COO-, -CONH-, -CO-, -S-, -C°C-, -CH=CH-, -CH=N-, -CH₂-, -N=N- und -N=N(O)- sind und R⁶-X⁵-R⁷ oder R⁶-X⁵-R⁷-X⁶ auch eine C-C-Bindung sein kann, wobei n in A¹ bzw. A² eine ganze Zahl von 1 bis 20 ist, wenn R¹ bzw. R⁴ -O- ist, und
- X⁴, X⁵ und X⁶ gleiche oder verschiedene Reste aus der Gruppe 1,4-Phenylen, 1,4-Cyclohexylen, mit B¹, B² und/oder B³ substituiertes Arylalkan oder Heteroarylalkan, das 1 bis 3 Heteroatome aus der Gruppe O, N und S enthält, mit 6 bis 10 Atomen im Arylkern und mit B¹, B² und/oder B³ substituiertes Cycloalkylen mit 3 bis 10 Kohlenstoffatomen sind, worin B¹, B² und B³ gleiche oder verschiedene Substituenten aus der Gruppe -H, C₁-C₂₀-Alkyl, C₁-C₂₀-Alkoxy, C₁-C₂₀-Alkylthio, C₁-C₂₀-Alkylcarbonyl, C₁-C₂₀-Alkoxycarbonyl, C₁-C₂₀-Alkylthiocarbonyl, -OH, Halogen (Fluor, Chlor, Brom, Iod), -CN, -NO₂, Cycloalkyl, Formyl und durch Ethersauerstoff, Thioetherschwefel oder Estergruppen unterbrochene Alkyl-, Alkoxy- oder Alkylthioreste mit 1-20 Kohlenstoffatomen sein können.

17. Verfahren nach Anspruch 16, dadurch gekennzeichnet, daß A³ und/oder A⁴ chirale Reste sind.

18. Verwendung der Verbindungen mit der allgemeinen Formel Y¹-A¹-M¹-A²-Y² gemäß einem der Ansprüche 1 bis 5 alleine oder im Gemisch mit Verbindungen der allgemeinen Formel Y³-A³-M²-A⁴-Y⁴ oder (Y³-A³)₂-M²-A⁴-Y⁴ gemäß einem der Ansprüche 16 oder 17 zur Herstellung von Effektlacken.
